# FASCICULE DE BREVET EUROPEEN

(11) **EP 1 773 914 B1**
(45) Date de publication et mention de la délivrance du brevet: **25.11.2009**
(21) Numéro de dépôt: 05792029.0
(22) Date de dépôt: 25.07.2005
(51) Int. Cl.: C08G 69/10, C08G 69/48, A61K 9/20, A61K 47/48, C08G 83/00

(54) **POLYAMINOACIDES FONCTIONNALISES PAR DES GREFFONS HYDROPHOBES PORTANT UNE CHARGE ANIONIQUE ET LEURS APPLICATIONS NOTAMMENT THERAPEUTIQUES**
MITTELS WASSERABWEISENDER IMPLANTATE MIT ANIONISCHER LADUNG FUNKTIONALISIERTE POLYAMINOSÄUREN UND ANWENDUNGEN, WIE ETWA THERAPEUTISCHE ANWENDUNGEN DAFÜR
POLYAMINO ACIDS FUNCTIONALISED BY HYDROPHOBIC GRAFTS BEARING AN ANIONIC CHARGE AND APPLICATIONS THEREOF, SUCH AS THERAPEUTIC APPLICATIONS

(30) Priorité: 30.07.2004 FR 0408478
(43) Date de publication de la demande: 18.04.2007
(73) Titulaire: FLAMEL TECHNOLOGIES, 69200 Vénissieux (FR)
(72) Inventeur: BREYNE, Olivier, F-69004 LYON (FR); POULIQUEN, Gauthier, F-69007 LYON (FR); CHAN, You-Ping, F-69008 LYON (FR)
(74) Mandataire: Fleurance, Raphaël
(86) Numéro de dépôt international: PCT/FR2005/050610
(87) Numéro de publication internationale: WO 2006/021710

(56) Documents cités:
- WO-A-00/30618
- WO-A-03/104303
- WO-A-2004/108796

## Description

La présente invention concerne des nouveaux matériaux à base de polyaminoacides biodégradables, utiles notamment pour la vectorisation de principe(s) actif(s) (PA).

L'invention vise aussi de nouvelles compositions pharmaceutiques, cosmétiques, diététiques ou phytosanitaires à base de ces polyaminoacides. Ces compositions peuvent être du type de celles permettant la vectorisation de PA et se présentant, de préférence, sous forme d'émulsions, de micelles, de particules, de gels, d'implants ou de films.

Les PA considérés sont, avantageusement, des composés biologiquement actifs et qui peuvent être administrés à un organisme animal ou humain par voie orale, parentérale, nasale, vaginale, oculaire, sous-cutanée, intraveineuse, intra-musculaire, intradermique, intrapéritonéale, intracérébrale, buccale, etc.

Les PA plus particulièrement, mais non limitativement, concernés par l'invention sont des protéines, des glycoprotéines, des peptides, des polysaccharides, des lipopolysaccharides, des oligo ou des polynucléotides, et des molécules organiques. Mais il peut aussi s'agir de produits cosmétiques ou de produits phytosanitaires, tels que des herbicides, des insecticides, des fongicides, etc.

Dans le domaine de la vectorisation des principes actifs notamment médicamenteux, il existe un besoin, dans beaucoup de cas :
- de les protéger contre la dégradation (hydrolyse, précipitation sur site, digestion enzymatique etc..) jusqu'à ce qu'ils atteignent leur site d'action,
- et/ou de contrôler leur vitesse de libération afin de maintenir un niveau thérapeutique sur une durée définie,
- et/ou de les véhiculer (en les protégeant) au site d'action.

A ces fins, plusieurs types de polymères ont été étudiés et certains sont même disponibles commercialement. On peut citer, par exemple, les polymères du type polylactique, polylactique-glycolique, polyoxyethylène-oxypropylène, polyaminoacide ou encore polysaccharide. Ces polymères constituent des matières premières permettant de fabriquer, par exemple, des implants massiques, des microparticules, des nanoparticules, des vésicules, des micelles ou des gels. Outre le fait que ces polymères doivent être adaptés à la fabrication de tels systèmes, ils doivent également être biocompatibles, non-toxiques, non-immunogènes, économiques et ils doivent pouvoir être facilement éliminés du corps et/ou être biodégradables. Sur ce dernier aspect, il est de surcroît essentiel que la biodégradation dans l'organisme génère des produits non-toxiques.

Un autre aspect très important dans le développement d'un polymère associatif est sa solubilité dans l'eau. La possibilité de solubiliser une quantité élevée de polymère permet d'avoir un rapport polymère/principe actif adapté au profil de libération souhaité.

A titre d'illustration de l'art antérieur concernant des polymères employés comme matières premières pour la réalisation de systèmes de vectorisation de PA, divers brevets ou demandes de brevet ou articles scientifiques sont évoqués ci-après.

Le brevet US-B-4,652,441 décrit des microcapsules de polylactide encapsulant l'hormone LH-RH. Ces microcapsules sont produites en préparant une émulsion eau-dans-huile-dans-eau et comprennent une couche interne aqueuse contenant l'hormone, une substance (gélatine) fixant cette dernière, une couche huileuse de polylactide, ainsi qu'une couche externe aqueuse (alcool polyvinylique). La libération du PA peut se faire sur une période de plus de deux semaines après injection sous-cutanée.

Le brevet US-B-6,153,193 décrit des compositions à base de micelles de poly(oxyéthylène)-poly(oxypropylène) amphiphiles, pour la vectorisation d'anti-cancéreux tel que l'adriamycine.

Akiyoshi et al. (J. Controlled Release 1998, 54, 313-320) décrivent des pullulans qui sont rendus hydrophobes par greffage de cholestérol et qui forment des nanoparticules dans l'eau. Ces nanoparticules aptes à se complexer de manière réversible avec l'insuline, forment des suspensions colloïdales stables.

Le brevet US-B-4,351,337 décrit des copolyaminoacides amphiphiles, à base de leucine et de glutamate, utilisables sous forme d'implants ou de microparticules pour la libération contrôlée de principes actifs. La libération de ces derniers peut se faire sur une durée très longue dépendant de la vitesse de dégradation du polymère.

Le brevet US-B-4,888,398 décrit des polymères à base de polyglutamate ou polyaspartate, et éventuellement polyleucine, avec des groupements pendants de type alkyloxycarbonylméthyle, placés de façon aléatoire sur la chaîne polyaminoacide. Ces polyaminoacides, greffés par des groupements latéraux e.g. méthoxycarbonylméthyle, sont utilisables sous forme d'implants biodégradables contenant un PA à libération prolongée.

Le brevet US-B-5,904,936 décrit des nanoparticules obtenues à partir d'un polymère bloc polyleucine-polyglutamate, aptes à former des suspensions colloïdales stables et capables de s'associer spontanément avec des protéines biologiquement actives sans les dénaturer. Ces dernières peuvent ensuite être libérées in vivo de manière contrôlée, sur une longue période.

Le brevet US-B-5,449,513 décrit des copolymères bloc amphiphiles comprenant un bloc polyoxyéthylène et un bloc polyaminoacide, par exemple poly(béta-benzyl-L-aspartate). Ces polymères polyoxyéthylène-polybenzylaspartate forment des micelles qui sont aptes à encapsuler des molécules actives hydrophobes telles que l'adriamycine ou l'indométhacine.

La demande de brevet WO-A-99/61512 décrit des polylysines et des polyornithines fonctionnalisées par un groupe hydrophobe (acide palmitique relié à la polylysine ou ornithine) et un groupe hydrophile (polyoxyéthylène). Ces polymères, par exemple la polylysine greffée avec des chaînes polyoxyéthylène et palmitoyle forment, en présence de cholestérol, des vésicules capables d'encapsuler la doxorubicine ou l'ADN. Ces polymères à base de polylysines sont cationiques en milieu physiologique.

Le brevet US-B-6,630,171 de la demanderesse, décrit des polymères blocs ou aléatoires poly(glutamate de sodium)-poly(glutamate de méthyle, d'éthyle, d'hexadécyle ou de dodécyle), aptes à former des suspensions colloïdales stables et capables de s'associer spontanément avec des protéines biologiquement actives sans les dénaturer. Ces dernières peuvent ensuite être libérées in vivo de manière contrôlée, sur une longue période. Ces copolyaminoacides linéaires amphiphiles sont modifiés par la présence d'une chaîne latérale alkyle hydrophobe. Ces groupements alkyles sont greffés de façon covalente sur le polymère via une fonction ester.

Dans le même domaine, la demanderesse a décrit dans plusieurs demandes de brevets des polymères à base de polyglutamate avec des concepts apparentés.
La demande WO-A-03/104303 décrit des polyaminoacides anioniques fonctionnalisés par de l'alpha-tocophérol. La demande WO-A-04/013206 décrit des polyaminoacides anioniques comportant des groupements hydrophobes et **caractérisés en ce que** ces groupements sont reliés au polymère par l'intermédiaire d'une rotule contenant deux fonctions amides, et plus précisément via un espaceur de type lysine ou ornithine. La demande PCT/FR03/03458 non publiée décrit des polyaminoacides fonctionnalisés par au moins un groupement oligoaminoacide à base de leucine et/ou isoleucine et/ou valine et/ou phénylalanine.

La demande de brevet WO-A-87/03891 (US-B-4,892,733) décrit des polyglutamates ou polyaspartates porteurs de groupements diacides de type malonique, succinique ou glutarique, liés à la chaîne polyaminoacide via une rotule d'espacement ("espaceur") de nature oligopeptidique. La présence du groupement diacide permet de fixer des cations calcium ou de former des anhydrides cycliques susceptibles de réagir avec un principe actif. Ces polymères sont utilisables notamment sous forme d'implants pour la libération lente in vivo d'un principe actif.

Ainsi, même si de très nombreuses solutions techniques ont été développées et proposées dans l'art antérieur pour la vectorisation des principes actifs médicamenteux, la réponse à l'ensemble des exigences est difficile à obtenir et demeure non satisfaisante. Plus spécifiquement, on a pu identifier un besoin non satisfait en un matériau biodégradable pour la réalisation de particules de vectorisation de principes actifs, ce matériau devant être capable de former une suspension aqueuse de nano- ou microparticules de vectorisation propres à s'associer réversiblement à des principes actifs et dans lequel l'une des améliorations recherchées serait d'avoir un rapport polymère/principe actif le plus élevé possible.

Dans ce contexte, l'un des objectifs essentiels de la présente invention est de fournir de nouveaux polyaminoacides, amphiphiles, linéaires et anioniques à pH physiologique animal (par exemple de l'ordre de 7,4), qui représentent un perfectionnement par rapport à ceux décrits dans les brevets ou demandes de brevet US-B-6,630,171, WO-A-03/104303, WO-A-04/013206 et PCT/FR03/03458 (non publiée) notamment en termes de formulation (association réversible) d'un principe actif tel qu'une protéine thérapeutique.

Un autre objectif essentiel de la présente invention est que ces polymères soient aptes à être utilisés pour la vectorisation de PA et permettent de satisfaire de manière optimale à tout ou partie des spécifications du cahier des charges, à savoir notamment :
○ capacité :
   ■ à former aisément et économiquement des suspensions colloïdales aqueuses stables,
   ■ à s'associer facilement avec de nombreux principes actifs,
   ■ et à libérer ces principes actifs in vivo,
○ biocompatibilité,
○ biodégradabilité.

Ces objectifs, parmi d'autres, sont atteints par la présente invention qui concerne tout d'abord un polyaminoacide amphiphile, linéaire ou branché, comprenant des unités acide aspartique et/ou des unités acide glutamique anioniques et comportant des greffons hydrophobes, tout ou partie de ces greffons étant anioniques ou "anionisables".

Il est du mérite de la demanderesse d'avoir eu l'idée, de façon tout à fait judicieuse et avantageuse, de fonctionnaliser des polyaminoacides de type poly(Asp) et/ou (Glu), biodégradables, avec des greffons hydrophobes possédant des charges anioniques et/ou avec des greffons hydrophobes "anionisables".

En d'autres termes, l'invention se rapporte à un polyaminoacide comprenant des unités acide aspartique et/ou des unités acide glutamique, dont certaines sont porteuses d'un ou de plusieurs greffons hydrophobes, identiques ou différents entre eux,
**caractérisé :**
**■ en ce que** les greffons hydrophobes répondent à la formule générale (**I)** suivante:
   (**I**) -X-(GH)-Y,

      dans laquelle
      → -X- est un motif de liaison entre la chaîne polyaminoacide et -GH-Y;
      → -GH- est un groupement hydrophobe et
      → -Y est un groupement présentant au moins une charge anionique et/ou une ou plusieurs fonctions ionisables identiques ou différentes entre elles et capables de donner chacune naissance à au moins une charge anionique, et
■ en ce que -GH- est exempt de reste d'alpha aminoacide.

Avantageusement, -Y peut être exempt de groupements diacides de type malonique succinique ou glutarique.

Ce polyaminoacide comprend des chaînes principales (ou squelettes) constituées au moins en partie par des unités acide aspartique et/ou des unités acide glutamique. En pratique, le polyaminoacide est essentiellement formé par ces unités monomèriques aspartiques et/ou glutamiques.

Une unité Asp ou Glu peut être greffée par un ou deux (en pratique un seul) greffons hydrophobes, anioniques ou "anionisables".

Ces nouveaux polymères poly(Glu) et/ou (Asp) amphiphiles se sont avérés être particulièrement bien adaptés notamment pour la vectorisation des protéines. De façon surprenante, il a été constaté que ces nouveaux polymères peuvent être mis en solution aqueuse à des concentrations bien plus élevées qu'avec des polymères analogues de l'art antérieur. Cette propriété est un atout pour pouvoir réaliser, selon les besoins, une formulation dotée d'un rapport polymère / principe actif plus élevé.

Au sens de l'invention :
➢ le terme "*polyaminoacide*" couvre, d'une part les PAA comportant un seul type d'unité "acide aminé" (par exemple soit des unités Glu (acide glutamique ou glutamate), soit des unités Asp (acide aspartique ou aspartate), soit un copolyaminoacide (comportant un mélange d'unités d'acides amines Glu et Asp dans un enchaînement de type aléatoire, gradient ou bloc) et, d'autre part, aussi bien les oligoaminoacides comprenant de 2 à 20 unités "acide aminé" que les polyaminoacides comprenant plus de 20 unités "acide aminé";
➢ le terme *"unité acide aminé"* vise un motif monomérique ou non formé par un squelette d'un acide aminé donné, quelles que puissent être les substitutions, pour autant qu'elles ne modifient pas la nature de l'acide aminé concerné.

Ces polymères présentent des propriétés surprenantes de fluidité, d'association et/ou d'encapsulation avec un ou plusieurs principes actifs, en comparaison avec des produits analogues.
Au sens de l'invention et dans tout le présent exposé, les termes "association" ou "associer" employés pour qualifier les relations entre un ou plusieurs principes actifs et les polyaminoacides, signifient en particulier que le ou les principes actifs sont liés au(x) polyaminoacide(s) notamment par une liaison faible, par exemple par liaison ionique et/ou par contact hydrophobe et/ou sont encapsulés par le ou les polyaminoacides.

Un autre avantage notable des polyaminoacides selon l'invention est qu'ils sont facilement enzymatiquement dégradés ou dégradables, en catabolites/métabolites non toxiques (acides aminés).

Le motif -X- de liaison du greffon hydrophobe (I) -X-GH-Y peut également être qualifié de motif d'espacement ("spacer") ou de rotule, permettant de relier le groupement hydrophobe GH à une chaîne principale du polyaminoacide. -X- peut comprendre, e.g. au moins une liaison covalente directe et/ou au moins une liaison amide et/ou au moins une liaison ester. Par exemple, -X- peut être un motif du type de ceux appartenant au groupe comportant : les unités "acide aminé" différentes de l'unité monomérique constitutive du polyaminoacide, les dérivés des aminoalcools, les dérives des diamines, les dérivés des diols et les dérivés des hydroxyacides.

Le greffage des greffons hydrophobes (I) -X-GH-Y sur la chaîne polyaminoacide peut se faire de diverses manières. On peut :
a) soit tout d'abord greffer -X- sur la chaîne polyaminoacide ou utiliser les fonctions réactives pendantes de la chaîne principale polyaminoacide à titre de rotule -X-, puis fixer -GH- sur les -X- de la chaîne polyaminoacide et enfin -Y sur les GH,
b) soit tout d'abord greffer -X- sur la chaîne polyaminoacide ou utiliser les fonctions réactives pendantes de la chaîne principale polyaminoacide à titre de rotule -X- puis préassembler un bloc -GH-Y ou mettre en oeuvre un bloc -GH-Y préexistant en tant que tel, lequel -GH-Y est ensuite greffé sur les -X- de la chaîne polyaminoacide,
c) soit préassembler un bloc -X-GH-Y qui est ensuite greffé sur la chaîne polyaminoacide, soit préassembler un bloc -X-GH ou mettre en oeuvre un bloc -X-GH préexistant en tant que tel lequel -X-GH est ensuite greffé la chaîne polyaminoacide et enfin -Y sur les GH.

La voie b) est privilégiée en pratique en utilisant les fonctions réactives pendantes de la chaîne principale polyaminoacide à titre de rotule -X- et en mettant en oeuvre un bloc -GH-Y préexistant en tant que tel, lequel -GH-Y étant ensuite greffé sur les -X- de la chaîne polyaminoacide.
Le greffage des GH est explicité plus en détail ci-après dans la description du procédé d'obtention des polyaminoacides selon l'invention.
Classiquement, les précurseurs des -GH-Y ou des -GH sont, par exemple, choisis dans le groupe comprenant les alcools et les amines, ces composés pouvant être fonctionnalisés facilement par l'homme de l'art.

Suivant une variante préférée de l'invention, le polyaminoacide est **caractérisé en ce que** -X- et/ou -Y est (sont) exempt(s) de reste(s) d'alpha aminoacide(s). De manière plus préférée encore -X-, -GH- et -Y sont exempts d'alpha aminoacides.

Suivant une caractéristique préférée, le groupement hydrophobe GH du greffon hydrophobe comporte de 8 à 30 atomes de carbone.

De préférence, la (ou les) fonction(s) ionisable(s) de -Y capable(s) de donner naissance à au moins une charge anionique est (sont) choisie(s) dans le groupe de fonctions comprenant : la fonction carboxylique/carboxylate, la fonction sulfonique/sulfonate, la fonction sulfurique/sulfate et la fonction phosphorique/phosphate.

De manière plus préférée encore, les groupements hydrophobes GH sont choisis dans le groupe comprenant :
- les alkyles linéaires ou ramifiés en C8 à C30 pouvant comporter éventuellement au moins une insaturation et/ou au moins un hétéroatome,
- les alkylaryles ou arylalkyles en C8 à C30 pouvant comporter éventuellement au moins une insaturation et/ou au moins un hétéroatome,
- et les (poly)cycliques en C8 à C30 pouvant comporter éventuellement au moins une insaturation et/ou au moins un hétéroatome.

Le polyaminoacide selon l'invention est préférablement l'un de ceux comprenant des unités d'alpha-L-glutamate et/ou d'acide alpha-L-glutamique ou des unités d'alpha-L-aspartate et/ou d'acide alpha-L-aspartique.

Plus précisément, les polyaminoacides selon la présente invention sont, par exemple, des homooligomères ou des homopolymères comprenant des unités d'alpha-L-glutamate et/ou d'acide alpha-L-glutamique ou des unités d'alpha-L-aspartate et/ou d'acide alpha-L-aspartique.

De manière plus préférée, les polyaminoacides selon l'invention répondent à la formule générale (II) suivante : 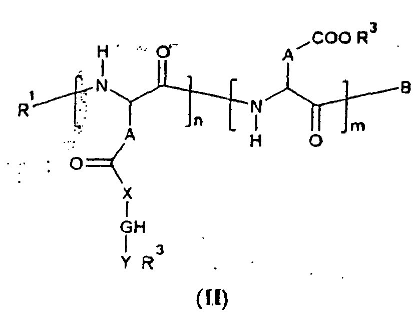 dans laquelle :
■ R¹ représente un H, un groupe acyle linéaire en C2 à C10 ou ramifié en C3 à C10 ou un pyroglutamate ;
■ A représente indépendamment un -CH₂- (unité acide aspartique) ou -CH₂-CH₂-(unité acide glutamique) ;
■ B représente :
   - un OR³, R³ répondant à la définition donnée ci-dessous,
   - un groupement NHR² dans laquelle R² représente un H, un alkyle linéaire en C2 à C10 ou ramifié en C3 à C10 ou un benzyle,
   - une unité acide aminé terminale liée par l'azote et dont la (les) fonction(s) acide(s) est (sont) éventuellement modifiée par une amine ou un alcool répondant aux définitions NHR² et OR² respectivement.
■ R³ est un H ou une entité cationique, de préférence sélectionnée dans le groupe comprenant :
   - les cations métalliques avantageusement choisis dans le sous-groupe comprenant : le sodium, le potassium, le calcium, le magnésium ;
   - les cations organiques avantageusement choisis dans le sous-groupe comprenant :
      - les cations à base d'amine,
      - les cations à base d'oligoamine,
      - les cations à base de polyamine (la polyéthylèneimine étant particulièrement préférée),
      - les cations à base de reste(s) d'acide(s) aminé(s) avantageusement choisis dans la classe comprenant les cations à base de lysine ou d'arginine,
   - ou les polyaminoacides cationiques avantageusement choisis dans le sous-groupe comprenant la polylysine ou l'oligolysine;
■ -X- est un motif de liaison -O-, -NH-, -N-alkyle- (C1 à C5), un résidu d'acide aminé (de préférence naturel), un diol, de diamine, d'aminoalcool
ou d'hydroxyacide comportant de 1 à 6 atomes de carbone;
■ -GH- représente un groupement hydrophobe comportant 8 à 30 atomes de carbone, de préférence choisi dans le groupe comprenant :
   - alkyles linéaires ou ramifiés en C8 à C30 pouvant comporter éventuellement au moins une insaturation et/ou au moins un hétéroatome (de préférence O et/ou N et/ou S), ou
   - alkylaryles ou arylalkyle en C8 à C30 pouvant comporter éventuellement au moins une insaturation et/ou au moins un hétéroatome (de préférence O et/ou N et/ou S), ou
   - (poly)cycliques en C8 à C30 pouvant comporter éventuellement au moins une insaturation et/ou au moins un hétéroatome (de préférence O et/ou N et/ou S);
■ -Y est:
   - une fonction "anionisable" constituée par une fonction carboxylique, sulfonique, sulfurique ou phosphorique (R³ est H), ou
   - Y est une fonction anionique constituée de préférence par une fonction carboxylate, sulfonate, sulfate ou phosphate (R³ est différent de H);
■ n/(n+m) est défini comme le taux de greffage molaire et varie de 0,5 à 100 % molaire ;
■ n + m varie de 3 à 1000, de préférence entre 30 et 500.

En pratique, au moins l'un des greffons hydrophobes de formule (**I**) : -X-GH-Y est, par exemple, choisi dans le groupe comprenant les espèces suivantes:

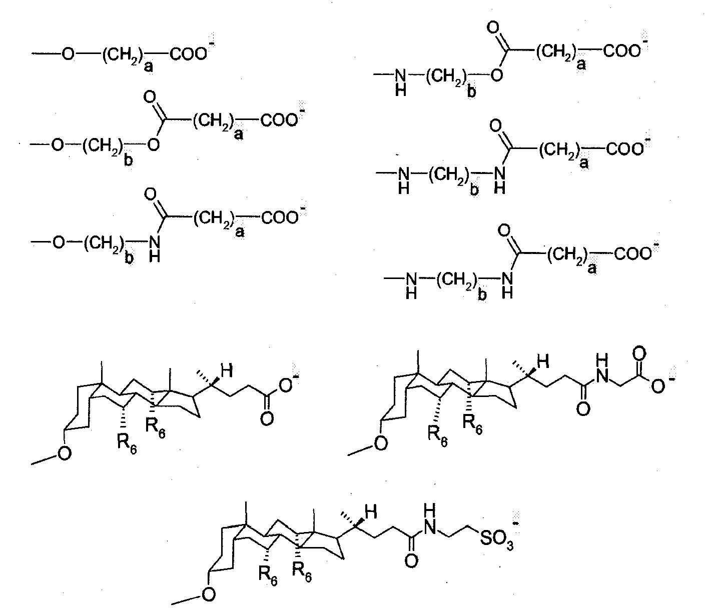

Dans ces structures :
a est compris entre 7 et 19,
b est compris entre 2 et 4,
R₆ est un H ou OH.

Selon une variante de l'invention, les polyaminoacides qu'elle concerne, sont non seulement porteurs de greffons hydrophobes ayant une charge anionique Y ou une fonction Y ionisable en anion, mais peuvent être également, porteurs d'au moins un greffon hydrophobe non ionisable. En particulier, le polyaminoacide peut être porteur d'au moins un greffon hydrophobe de formule **(III)** : -X'-GH', exempt de groupement ionisé ou ionisable et dans lequel -X'- et -GH' répondent aux mêmes définitions que celles données ci-dessus pour -X- et -GH-.
Ainsi, le groupement GH' non ionisé ou non ionisable peut être, par exemple, dérivé d'un groupement choisi dans le groupe comprenant les espèces suivantes : l'octanol, le dodécanol, le tétradécanol, l'hexadécanol, l'octadécanol, l'oleylalcool, le tocophérol ou le cholestérol.

Selon un premier mode de réalisation de l'invention, les chaînes principales des polyaminoacides sont des homopolymères d'alpha-L-glutamate ou d'acide alpha-L-glutamique.

Selon un deuxième mode de réalisation de l'invention, les chaînes principales des polyaminoacides sont des homopolymères d'alpha-L-aspartate ou d'acide alpha-L-aspartique.

Selon un troisième mode de réalisation de l'invention, les chaînes principales des polyaminoacides sont des copolymères d'alpha-L-aspartate/alpha-L-glutamate ou d'acide alpha-L-aspartique/alpha-L-glutamique.

Avantageusement, la distribution des unités acide aspartique et/ou acide glutamique de la chaîne polyaminoacide principale est telle que les polymères ainsi constitués sont soit aléatoires, soit de type bloc, soit de type multibloc.

Selon un autre mode de définition, les polyaminoacides selon l'invention ont une masse molaire qui se situe entre 2 000 et 200 000 g/mole, et de préférence entre 5 000 et 50 000 g/mole.

Il est par ailleurs préférable que le taux de greffage molaire en greffons hydrophobes des polyaminoacides selon l'invention, soit compris entre 2 et 100 %, et de préférence entre 5 et 50 %.

Selon des variantes, les polyaminoacides selon l'invention peuvent être linéaires, ou branchés.

Selon d'autres variantes, les polyaminoacides selon l'invention peuvent être porteurs d'au moins un greffon de type polyéthylène glycol lié à une unité glutamate et/ou aspartate.

Naturellement, l'invention couvre également des mélanges de polyaminoacides tels que définis ci-dessus.

De manière remarquable, les polyaminoacides de l'invention sont susceptibles d'être utilisés de plusieurs façons selon la nature des groupements hydrophobes et le degré de polymérisation du polyaminoacide. Les méthodes de mise en forme d'un polymère pour l'encapsulation d'un principe actif sous les diverses formes visées par l'invention sont connues de l'homme de l'art. Pour plus de détails, on peut se référer, par exemple à ces quelques références particulièrement pertinentes :
"Microspheres, Microcapsules and Liposomes ; vol 1. Preparation and chemical applications" Ed. R. Arshady, Citus Books 1999. ISBN : 0-9532187-1-6.
"Sustained-Release Injectable Products" Ed. J. Senior et M. Radomsky, Interpharm Press 2000. ISBN : 1-57491-101-5.
"Colloidal Drug Delivery Systems" Ed. J. Kreuter, Marcel Dekker, Inc. 1994. ISBN : 0-8247-9214-9.
"Handbook of Pharmaceutical Controlled Release Technology" Ed. D.L. Wise, Marcel Dekker, Inc. 2000. ISBN : 0-8247-0369-3.

Ces polyaminoacides sont en outre extrêmement intéressants du fait que, selon la longueur du polymère (degré de polymérisation) et la nature des groupements hydrophobes, ils se dispersent dans l'eau à pH 7,4 (par exemple avec un tampon phosphate) pour donner des solutions ou des suspensions colloïdales ou des gels structurés ou non, en fonction de la concentration en polymères. De plus, les polyaminoacides (sous forme de particules ou non), peuvent encapsuler ou s'associer aisément avec des principes actifs tels que des protéines, peptides ou petites molécules. La mise en forme préférée est celle décrite dans le brevet US-B-6,630,171 de la demanderesse et qui consiste à disperser le polymère dans l'eau et à incuber la solution en présence d'un principe actif (PA). Cette solution colloïdale de particules de vectorisation constituées des polyaminoacides selon l'invention, peut ensuite être filtrée sous 0,2 µm puis directement injectée à un patient.

Quand le rapport hydrophile/hydrophobe diminue, le polymère peut alors former des microparticules capables d'associer ou d'encapsuler des PA. Dans ce contexte, la mise en forme des microparticules peut se faire en co-solubilisant le PA et du polymère dans un solvant organique approprié puis le mélange précipité dans l'eau. Les particules sont ensuite récupérées par filtration et peuvent ensuite être utilisées pour une administration par voie orale (sous forme de gélule, sous forme compactée et/ou enrobée ou bien encore sous forme dispersée dans une huile) ou par voie parentérale après redispersion dans l'eau.

Selon une variante, le polymère peut être solubilisé dans un solvant biocompatible tel que la N-méthylpyrrolidone ou une huile appropriée telle que le Mygliol® puis injecté en intramusculaire ou sous-cutanée ou dans une tumeur. La diffusion du solvant ou de l'huile conduit à la précipitation du polymère sur le site d'injection et forme ainsi un dépôt. Ces dépôts assurent ensuite une libération contrôlée par diffusion et/ou par érosion et/ou par dégradation hydrolytique ou enzymatique du polymère.

Indépendamment du fait que la forme microparticulaire du polyaminoacide selon l'invention est préférée, les polymères de l'invention, sous forme neutre ou ionisée, sont de façon plus générale, utilisables seuls ou dans une composition liquide, solide ou gel et dans un milieu aqueux ou organique.

Il convient de comprendre que le polymère à base de polyaminoacides contient des fonctions qui sont soit neutres soit ionisées selon le pH et la composition. En solution aqueuse, le contre-cation peut être un cation métallique tel que le sodium, le calcium ou le magnésium, ou un cation organique tel que la triéthanolamine, le tris(hydroxyméthyl)-aminométhane ou une polyamine tels que la polyéthylèneimine.

Les polymères de l'invention sont par exemple obtenus par des méthodes connues de l'homme de l'art. Les polyaminoacides statistiques peuvent être obtenus par greffage du greffon hydrophobe formé par un -GH ou un -GH-Y préalablement fonctionnalisé par un motif de liaison -X- comportant par exemple au moins un acide aminé, directement sur le polymère par une réaction classique de couplage. Le motif de liaison -X- peut appartenir initialement à la chaîne principale du polyaminoacide. Les polyaminoacides blocs ou mulitiblocs peuvent être obtenus par polymérisation séquentielle des anhydrides de N-carboxyaminoacides (NCA) correspondants.

On prépare par exemple un polyaminoacide, homopolyglutamate, homopoly-aspartate ou un copolymère glutamate/aspartate, bloc, multibloc, aléatoire ou linéaire selon des méthodes classiques.
Pour l'obtention de polyaminoacide de type alpha, la technique la plus courante est basée sur la polymérisation d'anhydrides de N-carboxy-aminoacides (NCA), décrite, par exemple, dans l'article "Biopolymers, 1976, 15, 1869 et dans l'ouvrage de H.R. Kricheldorf "alpha-Aminoacid-N-carboxy Anhydrides and related Heterocycles" Springer Verlag (1987). Les dérivés d'NCA sont de préférence des dérivés NCA-O-Me, NCA-O-Et ou NCA-O-Bz (Me = Méthyl, Et = Ethyle et Bz = Benzyle). Les polymères sont ensuite hydrolysés dans des conditions appropriées pour obtenir le polymère sous sa forme acide. Ces méthodes sont inspirées de la description donnée dans le brevet FR-A-2 801 226 de la demanderesse. Un certain nombre de polymères utilisables selon l'invention, par exemple, de type acide poly(alpha-L-aspartique), acide poly(alpha-L-glutamique), acide poly(alpha-D-glutamique) et acide poly(gamma-L-glutamique) de masses variables sont disponibles commercialement. Le polymère polyaspartique de type alpha-bèta est obtenu par condensation de l'acide aspartique (pour obtenir un polysuccinimide) suivie d'une hydrolyse basique (cf. Tomida et al. Polymer 1997, 38, 4733-36).

Toujours à titre d'illustration non limitative, le couplage du greffon hydrophobe porteur de GH avec une fonction acide du polymère (motif de liaison -X-) peut être e.g. réalisé aisément par réaction du polyaminoacide, en présence d'un carbodiimide comme agent de couplage et optionnellement, un catalyseur tel que la 4-diméthylaminopyridine et dans un solvant approprié tel que le diméthylformamide (DMF), la N-méthyl pyrrolidone (NMP) ou le diméthylsulfoxide (DMSO). Le carbodiimide est, par exemple, le dicyclohexylcarbodiimide ou le diisopropylcarbodiimide. Le taux de greffage est contrôlé chimiquement par la stoechiométrie des constituants et réactifs ou le temps de réaction. Les greffons hydrophobes fonctionnalisés par un acide aminé (motif d'espacement/rotule) sont obtenus par couplage peptidique classique ou par condensation directe par catalyse acide. Ces techniques sont bien connues de l'homme de l'art.

Selon un autre de ses aspects, l'invention vise une composition pharmaceutique, cosmétique, diététique ou phytosanitaire comprenant au moins un polyaminoacide tel que défini ci-dessus et éventuellement au moins un principe actif, qui peut être notamment thérapeutique, cosmétique, diététique ou phytosanitaire.

Suivant une disposition intéressante de l'invention, le principe actif est associé au(x) polyaminoacide(s) par une ou plusieurs liaisons autre(s) qu'une (ou que des) liaison(s) chimique(s) covalente(s).
Les techniques d'association d'un ou de plusieurs PA aux polyaminoacides greffés selon l'invention, sont décrites notamment dans le brevet US-B-6,630,171. Elles consistent à incorporer au moins un principe actif dans le milieu liquide contenant des particules, de manière à obtenir une suspension colloïdale chargée en ou associée avec un ou plusieurs principe(s) actif(s) PA. Cette incorporation, qui conduit à un piégeage de PA par les particules, peut être réalisée de la manière suivante :
- mise en solution aqueuse de PA, puis ajout du polymère, soit sous forme de suspension colloïdale, soit sous forme de particules isolées (lyophilisat ou précipité) ;
- ou ajout de PA, soit en solution, soit à l'état pur ou préformulé, à une suspension colloïdale de particules, éventuellement préparée extemporanément par la dispersion de Particules de Vectorisation (PV) sèches dans un solvant approprié, tel que l'eau.

De préférence, le principe actif est une protéine, une glycoprotéine, une protéine liée à une ou plusieurs chaînes polyalkylèneglycol (de préférence PolyEthylèneGlycol (PEG) : "protéine-PEGylée"), un polysaccharide, un liposaccharide, un oligonucléotide, un polynucléotide ou un peptide.

Selon une variante, le principe actif est une "petite" molécule organique hydrophobe, hydrophile ou amphiphile.
Au sens du présent exposé, une "petite" molécule est notamment une petite molécule non protéinique.

Comme exemples de PA susceptibles d'être associés aux polyaminoacides selon l'invention, qu'ils soient ou non sous forme de (nano ou micro)particules, on peut citer :
○ les protéines telles que l'insuline, les interférons, les hormones de croissance, les interleukines, l'érythropoïétine ou les cytokines;
○ les peptides tels que la leuprolide ou la cyclosporine ;
○ les petites molécules telles que celles appartenant à la famille des anthracyclines, des taxoïdes ou des camptothécines ;
○ et leurs mélanges.

Selon un mode de réalisation, la composition de l'invention est sous forme d'un gel, d'une solution, d'une suspension, d'une émulsion, de micelles, de nanoparticules, de microparticules, d'un implant, d'une poudre ou d'un film.

Suivant l'une de ses formes particulièrement préférées, la composition, chargée ou non en principe(s) actif(s), est une suspension colloïdale stable de nanoparticules et/ou de microparticules et/ou de micelles de polyaminoacides, dans une phase aqueuse.

Selon une autre mode de réalisation, la composition de l'invention est sous forme de solution dans un solvant biocompatible et peut être injectée par voie sous-cutanée, intramusculaire ou dans une tumeur.

Selon une autre mode de réalisation, la composition peut éventuellement contenir un excipient pour l'ajustement du pH et/ou de l'osmolarité et/ou pour améliorer la stabilité (anti-oxydants) et/ou comme agent anti-microbiens. Ces excipients sont bien connus de l'homme de l'art (se référer à l'ouvrage : Injectable Drug Development, P.K. Gupta et al. Interpharm Press, Denver, Colorado 1999).

La composition selon l'invention, dès lors qu'elle est pharmaceutique, peut être administrée par voie orale, parentérale, nasale, vaginale, oculaire, sous-cutanée, intraveineuse, intramusculaire, intradermique, intrapéritonéale, intracérébrale ou buccale.

Il est également envisageable que la composition soit sous forme de solution dans un solvant biocompatible, susceptible d'être injectée en sous-cutané, intramusculaire ou dans une tumeur.
Selon une autre variante, la composition selon l'invention est formulée de telle sorte qu'elle soit apte à former un dépôt sur le site d'injection.

L'invention vise aussi des compositions qui comprennent des polyaminoacides selon l'invention et des principes actifs et qui sont susceptibles d'être utilisées pour la préparation :
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyEthylèneGlycol (PEG), on parle alors de protéines "PEGylées"}, des peptides, des polysaccharides, des liposaccharides, des oligonucléotides, des polynucléotides et des petites molécules organiques hydrophobes, hydrophiles
ou amphiphiles ;
- et/ou des nutriments,
- et/ou de produits cosmétiques ou phytosanitaires.

Selon encore un autre de ses aspects, l'invention vise un procédé de préparation :
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyEthylèneGlycol (PEG), on parle alors de protéines "PEGylées"}, des peptides, des polysaccharides, des liposaccharides, des oligonucléotides, des polynucléotides et des petites molécules organiques hydrophobes, hydrophiles
ou amphiphiles ;
- et/ou des nutriments ;
- et/ou de produits cosmétiques ou phytosanitaires ;
   ce procédé étant **caractérisé en ce qu**'il consiste essentiellement à mettre en oeuvre au moins un polyaminoacide tel que défmi ci-dessus et/ou la composition elle aussi décrite supra.

L'invention concerne également une méthode de traitement thérapeutique consistant essentiellement à administrer la composition telle que décrite dans le présent exposé, par voie orale, parentérale, nasale, vaginale, oculaire, sous-cutanée, intraveineuse, intramusculaire, intradermique, intrapéritonéale, intracérébrale ou buccale.

Suivant une variante particulière de l'invention, cette méthode de traitement thérapeutique consiste essentiellement à mettre la composition telle que décrite supra sous forme de solution dans un solvant biocompatible puis à l'injecter en sous-cutané, intramusculaire ou dans une tumeur, de préférence de manière à ce qu'elle forme un dépôt sur le site d'injection.

L'invention sera mieux comprise et ses avantages et variantes de mise en oeuvre ressortiront bien des exemples qui suivent et qui décrivent la synthèse des polyaminoacides, leur transformation en système de vectorisation de PA (suspension colloïdale aqueuse stable) et la démonstration de la capacité d'un tel système de s'associer à une protéine pour former des compositions pharmaceutiques.

### Exemple 1 : Synthèse du polymère pGluLC :

### Synthèse d'un polyglutamate (dont les fonctions réactives carboxylate latérale font office de motif de liaison -X-) greffé avec un groupement hydrophobe GH dérivé d'acide lithocholique

### 1/ Structure du précurseur du GH :

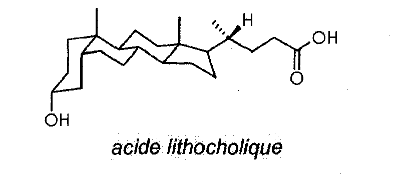

### Étape 1 : greffage des acides

Le polymère alpha-L-polyglutamique (de masse équivalente à environ 12 000 g/mole par rapport à un standard en polyoxyéthylène) est obtenu par polymérisation de monomères constitués par des dérivés N-CarboxyAnhydride de glutamate de méthyle : NCAGluOMe. Cette polymérisation est suivie d'une hydrolyse comme décrit dans la demande de brevet FR-A-2 801 226.
Dans un réacteur de 500 ml sous flux d'azote, 5 g de pGluOH sont solubilisés dans 90 ml de DMF à 80°C. La solution est agitée pendant 18 h à 80°C, puis refroidie à 15°C. Après l'ajout de 0,6 g de diisopropylcarbodiimide (DIPC), le mélange réactionnel est agité 30 min, puis 1,75 g d'acide lithocholique (LC) en solution dans 5 ml de DMF et 0,142 g de 4-diméthylaminopyridine (DMAP) en solution dans 3 ml de DMF sont successivement ajoutés. Le mélange réactionnel est agité 18 h à 15 °C, 5 ml d'HCl 1N sont ajoutés, puis le polymère est précipité en versant goutte à goutte le mélange réactionnel dans 400 ml d'eau salée (60 g NaCl) acide (pH = 2, HCl) et 200 ml d'éther diisopropylique. Après filtration sur verre fritté, le solide obtenu est lavé avec une solution d'HCl 0,1 N, puis un mélange eau/acétate d'éthyle. Cinq grammes d'un solide blanc sont obtenus après séchage dans une étuve sous vide.

Le taux de greffage mesuré par RMN du proton dans le TFA-*d* est de 7,2 %. Le Mn (déterminé par GPC NMP à 70 °C) est de 34 800 g/mol en équivalents PMMA.

### Étape 2 : neutralisation

Deux grammes du polymère de l'étape 1 sont mis en suspension dans 200 ml d'eau déminéralisée. On ajoute goutte à goutte de la soude 0,1N jusqu'à dissolution totale du solide, en prenant soin de ne pas dépasser pH = 9. Après neutralisation, le pH est ajusté à 7,4 avec une solution d'HCl 0,1N. Le Mn (déterminé par GPC aqueuse) est de 16 600 g/mol en équivalent POE.

### Exemple 2 : Synthèse du polymère pGluHLA :

### Synthèse d'un polyglutamate (dont les fonctions réactives carboxylate latérale font office de motif de liaison -X-) greffé avec le GH dérivé d'acide 12-hydroxylaurique (HLA)

### 1/ Structure du précurseur du GH :

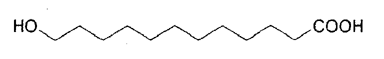

### Étape 1 : greffage des acides

Dans un réacteur de 500 ml sous flux d'azote, on solubilise 10 g de pGluOH (idem exemple 1) dans 180 ml de DMF à 80 °C et on agite pendant 18 h à cette température. On refroidit le mélange réactionnel à 15 °C, ajoute 3,91 g de DIPC et agite pendant 30 min. On additionne ensuite successivement 5,87 g de HLA en solution dans 15 ml de DMF et 0,57 g de DMAP en solution dans 5 ml de DMF. On agite à 15 °C pendant 6 h, ajoute 9 ml d'HCl 1N, puis précipite le polymère en versant goutte à goutte le mélange réactionnel dans 1 l d'eau acide (HCl) de pH = 2. On filtre sur verre fritté le solide blanc formé, puis le lave successivement avec une solution d'HCl 0,1 N, de l'eau, de l'acétate d'éthyle, et enfin de l'éther diisopropylique. Après séchage en étuve sous vide, on isole 12,6 g d'un solide blanc.
Le taux de greffage mesuré par RMN du proton dans le TFA-*d* est de 27,4 %. Le Mn (déterminé par GPC NMP à 70 °C) est de 38 000 g/mol en équivalents PMMA.

### Étape 2 : neutralisation

On suspend 4 g du polymère obtenu dans l'étape 1 dans 200 ml d'eau déminéralisée. On agite vigoureusement et ajoute goutte à goutte de la soude 0,1N jusqu'à dissolution totale du solide, en prenant soin de ne pas dépasser pH = 8. Après neutralisation, le pH est ajusté à 7,4 avec une solution d'HCl 0,1N. Le Mn (déterminé par GPC aqueuse) est de 18 600 g/mol en équivalent POE.

### Exemple 3 : Synthèse du polymère pGluHLA-T:

### Synthèse d'un polyglutamate (dont les fonctions réactives carboxylate latérale font office de motif de liaison -X-) greffé avec des GH dérivés d'acide 12-hydroxylaurique (HLA) et des GH' dérivés d'alpha-Tocophérol (T)

### 1/ Structures des précurseurs des GH :

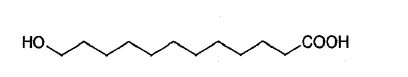

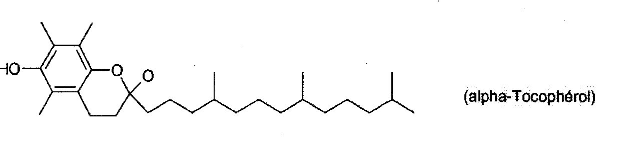

### Étape 1 : 1^{er} greffage des acides (avec la D, L-alpha-tocopherol d'origine synthétique)

Dans un réacteur de 500 ml sous flux d'azote, on solubilise 5 g de pGluOH (idem exemple 1) et 47 mg de DMAP dans 90 ml de DMF à 80 °C et on agite pendant 18 h à cette température. On refroidit le mélange réactionnel à 15 °C, ajoute successivement 1,67 g de D,L-alpha-tocophérol en solution dans 5 ml de DMF, 940 mg de DMAP en solution dans 5 ml de DMF, puis 780 mg de DIPC. On agite à 15 °C pendant 3 h30, ajoute 5 ml d'HCl 1N, puis précipite le polymère en versant goutte à goutte le mélange réactionnel dans 400 ml d'eau salée (60 g NaCl) acide (pH = 2, HCl) et 200 ml d'éther diisopropylique. On filtre sur verre fritté le solide blanc formé, puis le lave successivement 3 fois avec un mélange de 300 ml d'eau et 200 ml d'éther diisopropylique, puis 2 fois avec 300 ml d'éther diisopropylique. Après séchage en étuve sous vide, on isole 5,1 g d'un solide blanc.
Le taux de greffage mesuré par RMN du proton dans le TFA-*d* est de 9,5 %. Le Mn (déterminé par GPC NMP à 70 °C) est de 39 100 g/mol en équivalents PMMA.

### Étape 2 : 2^{e} greffage des acides (avec le HLA)

Dans un réacteur de 500 ml sous flux d'azote, on solubilise 5 g du polymère obtenu dans l'étape 1 dans 90 ml de DMF à 80 °C et on agite pendant 18 h à cette température. On refroidit le mélange réactionnel à 15 °C, ajoute 1,95 g de DIPC et agite pendant 30 min. On additionne ensuite successivement 3,35 g de HLA en solution dans 5 ml de DMF et 280 mg de DMAP en solution dans 5 ml de DMF. On agite à 15 °C pendant 2 h, puis à 20 °C pendant 4 h. On ajoute 5 mL d'HCl 1N, puis précipite le polymère en versant goutte à goutte le mélange réactionnel dans 600 ml d'eau acide (HCl) de pH = 2. On filtre sur verre fritté le solide blanc formé, le resolubilise dans 100 ml de DMF, puis le précipite à nouveau en versant goutte à goutte le mélange réactionnel dans 600 ml d'eau acide (HCl) de pH = 2. On filtre sur verre fritté le solide blanc formé, puis le lave successivement avec une solution d'HCl 0,1 N, de l'eau, et enfin de l'éther diisopropylique. Après séchage en étuve sous vide, on isole 5,6 g d'un solide blanc.
Le taux de greffage mesuré par RMN du proton dans le TFA-*d* est de 35 %. Le Mn (déterminé par GPC NMP à 70 °C) est de 43 000 g/mol en équivalents PMMA.

### Étape 3 : neutralisation

On suspend 2 g du polymère obtenu dans l'étape 2 dans 200 ml d'eau déminéralisée. On agite vigoureusement et ajoute goutte à goutte de la soude 1N jusqu'à dissolution totale du solide, en prenant soin de ne pas dépasser pH = 8. Après neutralisation, le pH est ajusté à 7,4 avec une solution d'HCl 0,1N. Le Mn (déterminé par GPC aqueuse) est de 12 900 g/mol en équivalent POE.

### Exemple 4 : Etudes des propriétés de solubilité et de viscosité

A titre de comparaison des propriétés et pour démontrer l'invention, 3 polymères de structures analogues ont été synthétisés selon les mêmes modes opératoires. Ces polymères ont les greffons suivants (sans aucune charge ionique) :
C1 : le cholestérol (CHOL)
C2 : le n-dodécanol (OC12)
C3 : l'alpha-tocophérol (T)

On réalise une mesure de la viscosité du polymère à pH 7,4 et à une osmolalité de 300 mOsmol en fonction de la concentration. On mesure ensuite la concentration limite d'agrégation Cη (g/l): concentration à partir de laquelle la viscosité augmente très rapidement. Les résultats sont rassemblés dans le tableau ci-dessous.

**TABLEAU 1**

| **Exemple** | **Polymère** | **% molaire du greffon** | **Cη (g/l)** |
|---|---|---|---|
| Exemple 1 | PgluLC | 7 % | 90 g/l |
| Exemple 2 | PGluHLA | 27 % | 150 g/l |
| Exemple 3 | PGluHLA-T | HLA : 35 %, T : 10 % | > 40 g/l |
| C1 | PgluChol | 5 % | 35 g/l |
| C2 | PGluOC12 | 20 % | 30 g/l |
| C3 | PgluT | 7 % | 20 g/l |

*C1 et C2 : polyglutamate selon le brevet* US-B-6,630,171
*C3 : polyglutamate selon le brevet* WO-A-03/104303

La comparaison des viscosités, illustrées par les valeurs Cη, montre qu'il est bien plus facile d'obtenir une solution concentrée avec les polymères de l'invention. Cette propriété permet donc de réaliser des formulations ayant des concentrations élevées en polymères pouvant augmenter ainsi le rapport polymère/principe actif, tout en assurant une bonne injectabilité.

### Exemple 5 : Etude d'association avec l'insuline

On prépare une solution aqueuse contenant une quantité définie de polymère par millilitre à pH 7,4 et 200 UI d'insuline (7,4 mg). On laisse incuber les solutions pendant deux heures à température ambiante et on sépare l'insuline libre de l'insuline associée par ultrafiltration (seuil à 100 KDa, 15 minutes sous 10000G à 18 °C). L'insuline libre récupérée dans le filtrat est ensuite dosée par CLHP (Chromatographie Liquide Haute Performance) et l'on déduit la quantité d'insuline associée. Les résultats sont donnés dans le tableau 2 ci-dessous.

**TABLEAU 2**

| Polymère | Concentration en polymère | % association |
|---|---|---|
| Ex 1 | 50 mg/ml | 82 % |
| Ex 3 | 50 mg/ml | 51 % |

Les résultats démontrent que les polymères de l'invention sont capables d'associer l'insuline pour donner des suspensions colloïdales de taille supérieure à 100 Kda et les taux d'association avec l'insuline sont très élevés. La capacité d'association de ces polymères les rend aptes à être utilisés comme agents de vectorisation.

## Revendications

1. Polyaminoacide comprenant des unités acide aspartique et/ou des unités acide glutamique, dont certaines sont porteuses d'un ou de plusieurs greffons hydrophobes, identiques ou différents entre eux,
**caractérisé :**
**■ en ce que** les greffons hydrophobes répondent à la formule générale (**I**) suivante:
(**I**) -X-(GH)-Y,
dans laquelle
→ -X- est un motif de liaison entre la chaîne polyaminoacide et -GH-Y;
→ -GH- est un groupement hydrophobe et
→ -Y est un groupement présentant au moins une charge anionique et/ou une ou plusieurs fonctions ionisables identiques ou différentes entre elles et capables de donner chacune naissance à au moins une charge anionique, et
■ **en ce que** -GH- est exempt de reste d'alpha aminoacide.

2. Polyaminoacide selon la revendication 1, **caractérisé en ce que** -X- et/ou -Y est (sont) exempt(s) de reste(s) d'alpha aminoacide(s).

3. Polyaminoacide selon l'une des revendications précédentes, **caractérisé en ce qu'**au moins un groupement hydrophobe -GH- comporte de 8 à 30 atomes de carbone.

4. Polyaminoacide selon l'une des revendications précédentes, **caractérisé en ce que** la (ou les) fonction(s) ionisable(s) de -Y capable(s) de donner naissance à au moins une charge anionique est (sont) choisie(s) dans le groupe de fonctions comprenant: la fonction carboxylique/carboxylate, la fonction sulfonique/sulfonate, la fonction sulfurique/sulfate et la fonction phosphorique/phosphate.

5. Polyaminoacide selon l'une des revendications précédentes **caractérisé en ce qu'**au moins un groupement hydrophobe GH est choisi dans le groupe comprenant :
■ les alkyles linéaires ou ramifiés en C8 à C30 pouvant comporter éventuellement au moins une insaturation et/ou au moins un hétéroatome,
■ les alkylaryles ou arylalkyles en C8 à C30 pouvant comporter éventuellement au moins une insaturation et/ou au moins un hétéroatome,
■ et les (poly)cycliques en C8 à C30 pouvant comporter éventuellement au moins une insaturation et/ou au moins un hétéroatome.

6. Polyaminoacide selon l'une des revendications précédentes, **caractérisé en ce que** sa chaîne principale comprend des unités d'alpha-L-glutamate et/ou d'alpha-L-glutamique ou des unités d'alpha-L-aspartate et/ou d'acide alpha-L-aspartique.

7. Polyaminoacide selon l'une des revendications précédentes **caractérisé par** la formule générale (**II**) suivante : 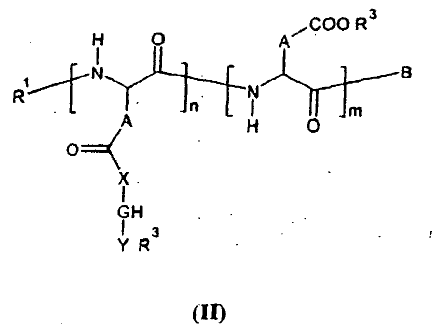 dans laquelle :
■ R¹ représente un H, un groupe acyle linéaire en C2 à C10 ou ramifié en C3 à C10 ou un pyroglutamate ;
■ A représente indépendamment un -CH₂- (unité acide aspartique) ou -CH₂-CH₂-(unité acide glutamique);
■ B représente :
- un OR³, R³ répondant à la définition donnée ci-dessous,
- un groupement NHR² dans lequel R² représente un H, un alkyle linéaire en C2 à C10 ou ramifié en C3 à C10 ou un benzyle,
- une unité acide aminé terminale liée par l'azote ;
■ R³ est un H ou une entité cationique, sélectionnée dans le groupe comprenant :
- les cations métalliques choisis dans le sous-groupe comprenant : le sodium, le potassium, le calcium, le magnésium ;
- les cations organiques choisis dans le sous-groupe comprenant :
• les cations à base d'amine,
• les cations à base d'oligoamine,
• les cations à base de polyamine,
• les cations à base de reste(s) d'acide(s) aminé(s) choisis dans la classe comprenant les cations à base de lysine ou d'arginine,
- ou les polyaminoacides cationiques choisis dans le sous-groupe comprenant la polylysine ou l'oligolysine;
■ -X- est un motif de liaison -O-, -NH-, -N-alkyle- (C1 à C5), un résidu d'acide aminé, un diol, une diamine, un aminoalcool ou un hydroxyacide;
■ -GH- représente un groupement hydrophobe comportant 8 à 30 atomes de carbone, choisi dans le groupe comprenant :
• les alkyles linéaires ou ramifiés en C8 à C30 pouvant comporter au moins une insaturation et/ou au moins un hétéroatome, ou
• les alkylaryles où les arylalkyle en C8 à C30 pouvant comporter au moins une insaturation et/ou au moins un hétéroatome, ou
• les (poly)cycliques en C8 à C30 pouvant comporter au moins une insaturation et/ou au moins un hétéroatome;
■ -Y est :
- une fonction "anionisable" constituée par une fonction carboxylique, sulfonique, sulfurique ou phosphorique (R³ est H), ou
- une fonction anionique constituée par une fonction carboxylate, sulfonate, sulfate ou phosphate (R³ est différent de H);
■ n/(n+m) est défini comme le taux de greffage molaire et varie de 0,5 à 100 % molaire ;
■ n + m varie de 3 à 1000.

8. Polyaminoacide selon l'une des revendications précédentes, **caractérisé en ce qu'**au moins l'un des greffons hydrophobes de formule (**I**) : -X-GH-Y est un radical choisi dans le groupe comprenant les espèces suivantes: 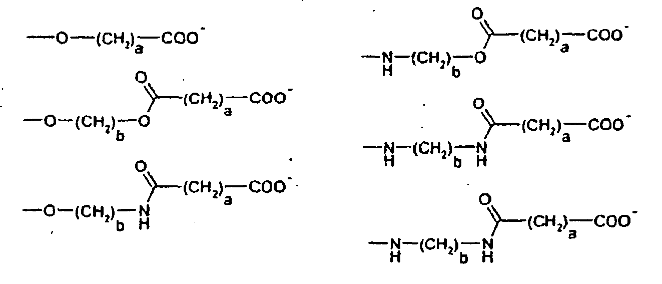 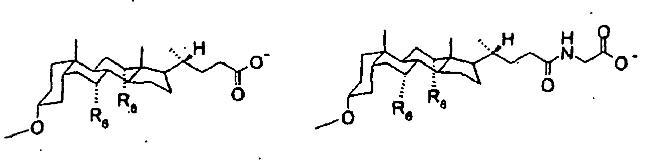 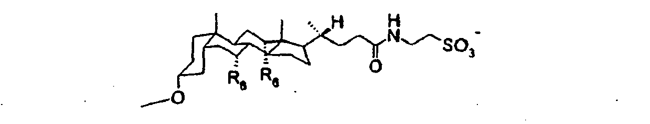 dans lesquelles:
■ a est compris entre 7 et 19,
■ b est compris entre 2 et 4,
■ R₆ représente un H ou un OH.

9. Polyaminoacide selon l'une des revendications précédentes, **caractérisé en ce qu'**il est porteur d'au moins un greffon hydrophobe de formule **(III) :** -X'-GH', exempt de groupement ionisé ou ionisable et dans lequel -X'- et -GH' répondent aux mêmes définitions que celles données pour -X- et -GH- dans au moins l'une des revendications 1, 2, 5-8.

10. Polyaminoacide selon la revendication 9, **caractérisé en ce que** le groupement hydrophobe GH' est un dérivé d'un groupement choisi dans le groupe comprenant les espèces suivantes: l'octanol, le dodécanol, le tétradécanol, l'hexadécanol, l'octadécanol, l'oleylalcool, le tocophérol ou le cholestérol.

11. Polyaminoacide selon l'une des revendications précédentes, **caractérisé en ce que** sa masse molaire se situe entre 2 000 et 200 000 g/mole.

12. Polyaminoacide selon l'une des revendications précédentes, **caractérisé en ce qu'**il est porteur d'au moins un greffon de type polyalkylène lié à une unité glutamate et/ou aspartate.

13. Composition pharmaceutique, cosmétique, diététique ou phytosanitaire comprenant au moins un polyaminoacide selon l'une quelconque des revendications 1 à 12.

14. Composition selon la revendication 13, **caractérisée en ce qu'**elle comprend au moins un principe actif.

15. Composition, selon la revendication 14, **caractérisée en ce que** le principe actif est associé au(x) polyaminoacide(s) par une ou plusieurs liaisons autre(s) qu'une (ou que des) liaison(s) chimique(s) covalente(s).

16. Composition selon la revendication 14 ou 15, **caractérisée en ce que** le principe actif est une protéine, une glycoprotéine, une protéine liée à une ou plusieurs chaînes polyalkylèneglycol, un polysaccharide, un liposaccharide, un oligonucléotide, un polynucléotide ou un peptide.

17. Composition selon l'une quelconque des revendications 13 à 16, **caractérisée en ce qu'**elle peut être administrée par voie orale, parentérale, nasale, vaginale, oculaire, sous-cutanée, intraveineuse, intramusculaire, intradermique, intra péritonéale, intracérébrale ou buccale.

18. Composition selon l'une quelconque des revendications 13 à 17, **caractérisée en ce qu'**elle est sous forme d'un gel, d'une solution, d'une émulsion, de micelles, de nanoparticules, de microparticules, d'une poudre ou d'un film.

19. Composition selon l'une quelconque des revendications 13 à 18, **caractérisée en ce qu'**elle est une suspension colloïdale de nanoparticules et/ou de microparticules et/ou de micelles de polyaminoacides, dans une phase aqueuse.

20. Composition selon l'une quelconque des revendications 13 à 19, **caractérisée en ce qu'**elle est sous forme de solution dans un solvant biocompatible et **en ce qu'**elle peut être injectée par voie sous-cutanée, intramusculaire ou dans une tumeur.

21. Composition selon la revendication 20, **caractérisée en ce qu'**elle est apte à former un dépôt sur le site d'injection.

22. Procédé de préparation :
• de médicaments, pour administration orale, nasale, vaginale, oculaire, sous-cutanée, intraveineuse, intramusculaire, intradermique, intrapéritonéale ou intracérébrale, les principes actifs de ces médicaments pouvant être des protéines, des glycoprotéines, des protéines liées à une ou plusieurs chaînes polyalkylèneglycol, des peptides, des polysaccharides, des liposaccharides, des oligonucléotides, des polynucléotides et des molécules organiques non protéiniques hydrophobes, hydrophiles ou amphiphiles ;
• et/ou des nutriments ;
• et/ou de produits cosmétiques ou phytosanitaires ;
**caractérisé en ce qu'**il consiste essentiellement à mettre en oeuvre au moins un polyaminoacide selon l'une quelconque des revendications 1 à 12 et/ou la composition selon l'une quelconque des revendications 13 à 21.

## Claims

1. Polyamino acid comprising aspartic acid units and/or glutamic acid units, certain of which bear one or more hydrophobic grafts, which are identical to or different from each other,
**characterized**
**■ in that** the hydrophobic grafts correspond to the following general formula **(I):**
**(I)** -X-(GH)-Y,
in which
→ -X- is a binding unit between the polyamino acid chain and -GH-Y;
→ -GH- is a hydrophobic group and
→ -Y is a group having at least one anionic charge and/or one or more ionizable functions which are identical to or different from each other and each capable of giving rise to at least one anionic charge, and
■ **in that** -GH- is free of alpha amino acid residue.

2. Polyamino acid according to claim 1, **characterized in that** -X- and/or -Y is (are) free of alpha amino acid residue(s).

3. Polyamino acid according to one of the previous claims, **characterized in that** at least one hydrophobic group -GH- comprises 8 to 30 carbon atoms.

4. Polyamino acid according to one of the previous claims, **characterized in that** the ionizable function(s) of -Y capable of giving rise to at least one anionic charge is (are) chosen from the group of functions comprising: the carboxylic/carboxylate function, the sulphonic/sulphonate function, the sulphuric/sulphate function and the phosphoric/phosphate function.

5. Polyamino acid according to one of the previous claims **characterized in that** at least one hydrophobic group GH is chosen from the group comprising:
■ the linear or branched C8 to C30 alkyls being able optionally to comprise at least one unsaturation and/or at least one heteroatom,
■ the C8 to C30 alkylaryls or arylalkyls being able optionally to comprise at least one unsaturation and/or at least one heteroatom,
■ and the C8 to C30 (poly)cyclics being able optionally to comprise at least one unsaturation and/or at least one heteroatom.

6. Polyamino acid according to one of the previous claims, **characterized in that** its main chain comprises alpha-L-glutamate and/or alpha-L-glutamic acid units or alpha-L-aspartate and/or alpha-L-aspartic acid units.

7. Polyamino acid according to one of the previous claims **characterized by** the following general formula **(II):** 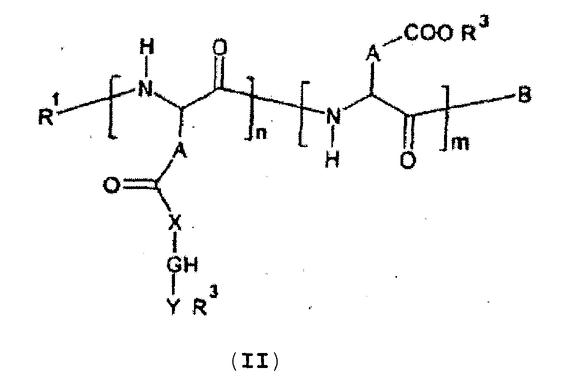 in which:
■ R¹ represents an H, a linear C2 to C10 or branched C3 to C10 acyl group, or a pyroglutamate;
■ A represents independently a -CH₂- (aspartic acid unit) or -CH₂-CH₂- (glutamic acid unit);
■ B represents
- an OR³, R³ corresponding to the definition given below,
- an NHR² group in which R² represents an H, a linear C1 to C10 or branched C3 to C10 alkyl or a benzyl,
- a terminal amino acid unit bound by the nitrogen;
■ R3 is an H or a cationic entity, selected from the group comprising:
- metallic cations chosen from the subgroup comprising: sodium, potassium, calcium, magnesium;
- organic cations advantageously chosen from the subgroup comprising:
• cations based on amine,
• cations based on oligoamine,
• cations based on polyamine,
• cations based on amino acid residue(s) chosen from the class comprising cations based on lysine or arginine,
- or cationic polyamino acids chosen from the subgroup comprising polylysine or oligolysine;
■ -X- is a binding unit -O-, -NH-, -N-alkyl-(C1 to C5), an amino acid residue, a diol, a diamine, an amino alcohol or a hydroxy acid;
■ -GH- represents a hydrophobic group comprising 8 to 30 carbon atoms, chosen from the group comprising:
• the linear or branched C8 to C30 alkyls being able to comprise at least one unsaturation and/or at least one heteroatom, or
• the C8 to C30 alkylaryls or arylalkyls being able to comprise at least one unsaturation and/or at least one heteroatom,
• the C8 to C30 (poly)cyclics being able to comprise at least one unsaturation and/or at least one heteroatom.
■ -Y is:
- an "anionizable" function constituted by a carboxylic, sulphonic, sulphuric or phosphoric function (R³ is H), or
- an anionic function constituted by a carboxylate, sulphonate, sulphate or phosphate function (R³ is different from H) ;
■ n/(n+m) is defined as the molar grafting rate and varies from 0.5 to 100% molar;
■ n + m varies from 3 to 1,000.

8. Polyamino acid according to one of the previous claims, **characterized in that** at least one of the hydrophobic grafts of formula **(I):** -X-GH-Y is a radical chosen from the group comprising the following species: 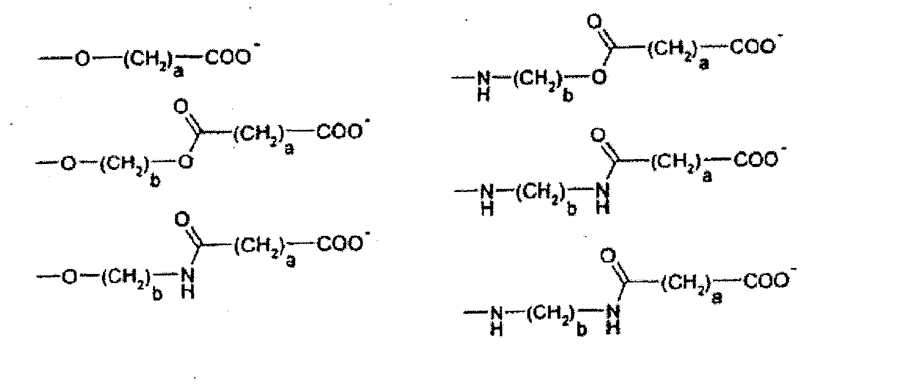 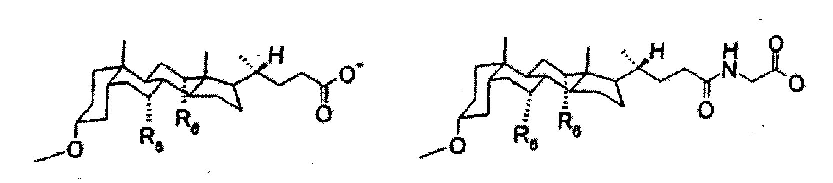 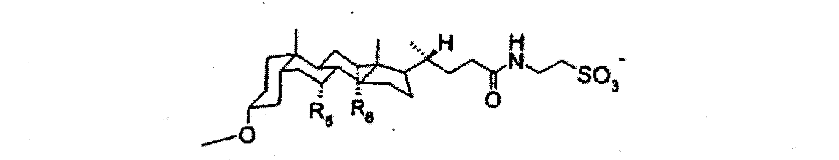 in which:
■ a is comprised between 7 and 19;
■ b is comprised between 2 and 4;
■ R₆ represents an H or an OH.

9. Polyamino acid according to one of the previous claims, **characterized in that** it bears at least one hydrophobic graft of formula **(III):** -X'-GH', free of an ionized or ionizable group in which -X'- and -GH' correspond to the same definitions as those given for -X- and -GH- in at least one of claims 1, 2, 5-8.

10. Polyamino acid according to claim 9, **characterized in that** the hydrophobic group GH' is derived from a group chosen from the group comprising the following species: octanol, dodecanol, tetradecanol, hexadecanol, octadecanol, oleyl alcohol, tocopherol or cholesterol.

11. Polyamino acid according to one of the previous claims, **characterized in that** its molar mass is situated between 2,000 and 200,000 g/mole.

12. Polyamino acid according to one of the previous claims, **characterized in that** it bears at least one graft of polyalkylene type bound to a glutamate and/or aspartate unit.

13. Pharmaceutical, cosmetic, dietetic or phytosanitary composition comprising at least one polyamino acid according to any one of claims 1 to 12.

14. Composition according to claim 13, **characterized in that** it comprises at least one active ingredient.

15. Composition, according to claim 14, **characterized in that** the active ingredient is combined with the polyamino acid(s) by one or more bonds other than one (or more) covalent chemical bond(s).

16. Composition according to claim 14 or 15, **characterized in that** the active ingredient is a protein, a glycoprotein, a protein bound to one or more polyalkylene glycol chains, a polysaccharide, a liposaccharide, an oligonucleotide, a polynucleotide or a peptide.

17. Composition according to any one of claims 13 to 16, **characterized in that** it can be administered by oral, parenteral, nasal, vaginal, ocular, sub-cutaneous, intravenous, intramuscular, intradermal, intraperitoneal, intracerebral or buccal route.

18. Composition according to any one of claims 13 to 17, **characterized in that** it is in the form of a gel, a solution, an emulsion, micelles, nanoparticles, microparticles, a powder or a film.

19. Composition according to any one of claims 13 to 18, **characterized in that** it is a colloidal suspension of nanoparticles and/or of microparticles and/or of micelles of polyamino acids, in an aqueous phase.

20. Composition according to any one of claims 13 to 19, **characterized in that** it is in the form of solution in a biocompatible solvent and **in that** it can be injected by sub-cutaneous or intramuscular route or into a tumour.

21. Composition according to claim 20, **characterized in that** it is capable of forming a deposit on the injection site.

22. Method for the preparation:
• of medicaments, for oral, nasal, vaginal, ocular, sub-cutaneous, intravenous, intramuscular, intradermal, intraperitoneal or intracerebral administration, the active ingredients of these medicaments being able to be proteins, glycoproteins, proteins bound to one or more polyalkylene glycol chains, peptides, polysaccharides, liposaccharides, oligonucleotides, polynucleotides and hydrophobic, hydrophilic or amphiphilic non-protein organic molecules;
• and/or of nutrients;
• and/or of cosmetic or phytosanitary products;
**characterized in that** it essentially consists of utilizing at least one polyamino acid according to any one of claims 1 to 12 and/or the composition according to any one of claims 13 to 21.

## Patentansprüche

1. Polyaminosäure, die Asparaginsäureeinheiten und/oder Glutaminsäureeinheiten umfaßt, von denen manche eine oder mehrere hydrophobe Pfropfe, die gleich oder verschieden voneinander sind, tragen,
**dadurch gekennzeichnet:**
■ **daß** die hydrophoben Propfe die allgemeine Formel (I) unten aufweisen:
(I) -X-(GH)-Y,
in der
→ -X- ein Bindungsmuster zwischen der Polyaminosäurekette und -GH-Y bedeutet;
→ -GH- eine hydrophobe Gruppe bedeutet und
→ -Y eine Gruppe bedeutet, die mindestens eine anionische Ladung und/oder eine oder mehrere gleiche oder verschiedene voneinander ionisierbare Funktionen, die jeweils fähig sind, mindestens eine anionische Ladung zu erzeugen, aufweist, und
■ **daß** -GH- frei von alpha-Aminosäureresten ist.

2. Polyaminosäure nach Anspruch 1, **dadurch gekennzeichnet, daß** -X- und/oder -Y frei von alpha-Aminosäureresten ist/sind.

3. Polyaminosäure nach einem der vorhergehenden Ansprüche, **dadurch gekennzeichnet, daß** mindestens eine hydrophobe -GH-Gruppe 8 bis 30 Kohlenstoffatome umfaßt.

4. Polyaminosäure nach einem der vorhergehenden Ansprüche, **dadurch gekennzeichnet, daß** die ionisierbare(n) Funktion(en) von -Y-, die fähig ist/sind, mindestens eine anionische Ladung zu erzeugen, aus der Gruppe der Funktionen, umfassend: Carbonsäure/Carboxylatfunktion, Sulfonsäure/Sulfonatfunktion, Schwefelsäure/Sulfatfunktion und Phosphorsäure/Phosphatfunktion, ausgewählt ist/sind.

5. Polyaminosäure nach einem der vorhergehenden Ansprüche, **dadurch gekennzeichnet, daß** mindestens eine hydrophobe GH-Gruppe aus der Gruppe, umfassend:
• lineare oder verzweigte C8- bis C30-Alkyle, die gegebenenfalls mindestens eine Unsättigung und/oder mindestens ein Heteroatom umfassen können,
• C8- bis C30-Alkylaryle oder -Arylalkyle, die gegebenenfalls mindestens eine Unsättigung und/oder mindestens ein Heteroatom umfassen können,
• sowie C8- bis C30-(Poly)zyklen, die gegebenenfalls mindestens eine Unsättigung und/oder mindestens ein Heteroatom umfassen können,
ausgewählt ist.

6. Polyaminosäure nach einem der vorhergehenden Ansprüche, **dadurch gekennzeichnet, daß** ihre Hauptkette alpha-L-Glutamat- und/oder alpha-L-Glutaminsäure- oder alpha-L-Aspartat- und/oder alpha-L-Asparaginsäureeinheiten umfaßt.

7. Polyaminosäure nach einem der vorhergehenden Ansprüche, die durch die folgende allgemeine Formel (II) **gekennzeichnet** ist: 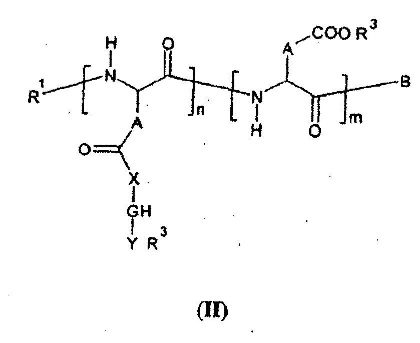 wobei:
■ R¹ für ein H, eine lineare C2- bis C10-Acylgruppe oder eine verzweigte C3- bis C10-Acylgruppe oder ein Pyroglutamat steht;
■ A unabhängig für ein -CH₂- (Asparaginsäureeinheit) oder -CH₂-CH₂- (Glutaminsäureeinheit) steht;
■ B:
- ein OR³, wobei R³ wie unten definiert ist,
- eine NHR²-Gruppe, in der R² ein H, ein lineares C2- bis C10-Alkyl oder ein verzweigtes C3- bis C10-Alkyl oder ein Benzyl bedeutet,
- eine über den Stickstoff gebundene terminale Aminosäureeinheit bedeutet;
■ R³ ein H oder eine kationische Einheit ist, die ausgewählt ist aus der Gruppe, umfassend:
- metallische Kationen, ausgewählt aus der Untergruppe, umfassend: Natrium, Kalium, Calcium, Magnesium;
- organische Kationen, ausgewählt aus der Untergruppe, umfassend:
• Kationen auf der Basis von Amin,
• Kationen auf der Basis von Oligoamin,
• Kationen auf der Basis von Polyamin,
• Kationen auf der Basis von Aminosäurerest(en), ausgewählt aus der Klasse, umfassend Kationen auf der Basis von Lysin oder Arginin,
- oder kationische Polyaminosäuren, ausgewählt aus der Untergruppe, umfassend Polylysin oder Oligolysin;
■ -X- ein Bindungsmuster -O-, -NH-, -N(C1- bis C5-)Alkyl, ein Aminosäurerest, ein Diol, ein Diamin, ein Aminoalkohol oder eine Hydroxysäure ist,
■ GH- eine hydrophobe Gruppe, umfassend 8 bis 30 Kohlenstoffatome, bedeutet, ausgewählt aus der Gruppe, umfassend:
• lineare oder verzweigte C8- bis C30-Alkyle, die mindestens eine Unsättigung und/oder mindestens ein Heteroatom umfassen können, oder
• C8- bis C30-Alkylaryle oder -Arylalkyle, die mindestens eine Unsättigung und/oder mindestens ein Heteroatom umfassen können, oder
• C8- bis C30-(Poly)zyklen, die mindestens eine Unsättigung und/oder mindestens ein Heteroatom umfassen können,
■ -Y bedeutet:
- eine "anionisierbare" Funktion, bestehend aus einer Carbonsäure-, Sulfonsäure-, Schwefelsäure- oder Phosphorsäurefunktion (R³ bedeutet H), oder
- eine anionische Funktion, bestehend aus einer Carboxylat-, Sulfonat-, Sulfat- oder Phosphatfunktion (R³ bedeutet nicht H);
■ n/(n+m) als molare Propfrate definiert ist und von 0,5 bis 100 Mol-% variiert;
■ n+m von 3 bis 1000 variiert.

8. Polyaminosäure nach einem der vorhergehenden Ansprüche, **dadurch gekennzeichnet, daß** mindestens einer der hydrophoben Pfropfe der Formel **(I)**: -X-GH-Y einen Rest bedeutet, ausgewählt aus der Gruppe, umfassend die folgenden Mitglieder: 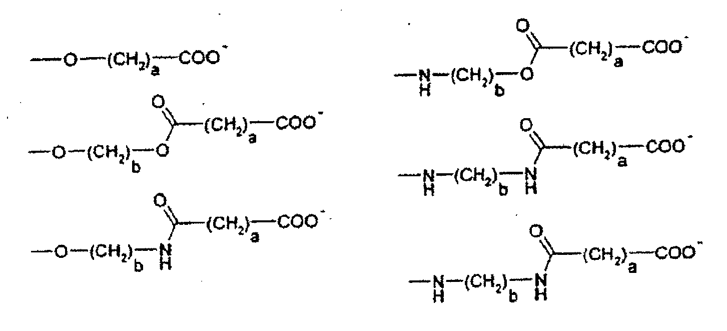 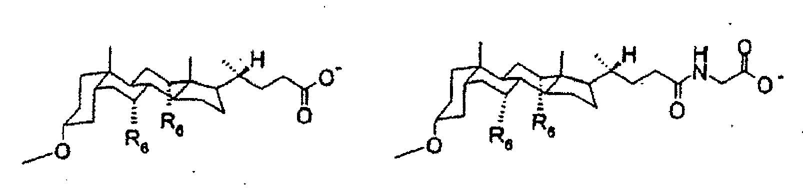 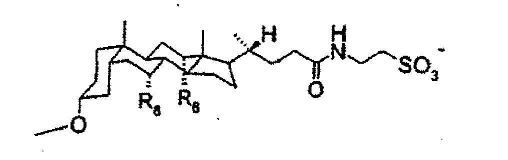 in denen:
■ a zwischen 7 und 19 ist,
■ b zwischen 2 und 4 ist,
■ R₆ H oder OH bedeutet.

9. Polyaminosäure nach einem der vorhergehenden Ansprüche, **dadurch gekennzeichnet, daß** sie mindestens einen hydrophoben Pfropf der Formel (**III**) trägt: -X'-GH', der frei von ionisierten oder ionisierbaren Gruppen ist und in dem -X'- und -GH' wie oben in mindestens einem der Ansprüche 1, 2, 5-8 für -X- und -GH- definiert sind.

10. Polyaminosäure nach Anspruch 9, **dadurch gekennzeichnet, daß** die hydrophobe GH'-Gruppe von einer Gruppe abstammt, ausgewählt aus der Gruppe, umfassend die folgenden Mitglieder: Octanol, Dodecanol, Tetradecanol, Hexadecanol, Octadecanol, Oleylalkohol, Tocopherol oder Cholesterol.

11. Polyaminosäure nach einem der vorhergehenden Ansprüche, **dadurch gekennzeichnet, daß** ihre Molmasse zwischen 2.000 und 200.000 g/mol liegt.

12. Polyaminosäure nach einem der vorhergehenden Ansprüche, **dadurch gekennzeichnet, daß** sie mindestens einen Pfropf des Polyalkylentyps, der an eine Glutamat- und/oder Aspartateinheit gebunden ist, trägt.

13. Pharmazeutische Zusammensetzung, kosmetische Zusammensetzung, diätetische Zusammensetzung oder Pflanzenschutzzusammensetzung, die mindestens eine Polyaminosäure nach einem der Ansprüche 1 bis 12 umfaßt.

14. Zusammensetzung nach Anspruch 13, **dadurch gekennzeichnet, daß** sie mindestens einen Wirkstoff umfaßt.

15. Zusammensetzung nach Anspruch 14, **dadurch gekennzeichnet, daß** der Wirkstoff mit der Polyaminosäure bzw. den Polyaminosäuren durch eine oder mehrere Bindungen verbunden ist, bei der bzw. denen es sich nicht um eine kovalente chemische Bindung bzw. nicht um kovalente chemische Bindungen handelt.

16. Zusammensetzung nach Anspruch 14 oder 15, **dadurch gekennzeichnet, daß** es sich bei dem Wirkstoff um ein Protein, ein Glykoprotein, ein an eine oder mehrere Polyalkylenglykolketten gebundenes Protein, ein Polysaccharid, ein Liposaccharid, ein Oligonukleotid, ein Polynukleotid oder eine Peptid handelt.

17. Zusammensetzung nach einem der Ansprüche 13 bis 16, **dadurch gekennzeichnet, daß** sie auf oralem, parenteralem, nasalem, vaginalem, okularem, subkutanem, intravenösem, intramuskulärem, intradermalem, intraperitonealem, intrazerebralem oder bukkalem Weg verabreicht werden kann.

18. Zusammensetzung nach einem der Ansprüche 13 bis 17, **dadurch gekennzeichnet, daß** sie in Form eines Gels, einer Lösung, einer Emulsion, von Mizellen, von Nanopartikeln, von Mikropartikeln, eines Pulvers oder eines Films vorliegt.

19. Zusammensetzung nach einem der Ansprüche 13 bis 18, **dadurch gekennzeichnet, daß** es sich um eine kolloidale Suspension von Nanopartikeln und/oder Mikropartikeln und/oder von Mizellen von Polyaminosäuren in einer wäßrigen Phase handelt.

20. Zusammensetzung nach einem der Ansprüche 13 bis 19, **dadurch gekennzeichnet, daß** sie in Form einer Lösung in einem biologisch kompatiblen Lösungsmittel vorliegt und daß sie auf subkutanem Weg, auf intramuskulärem Weg oder in einen Tumor injiziert werden kann.

21. Zusammensetzung nach Anspruch 20, **dadurch gekennzeichnet, daß** sie sich zur Bildung einer Ablagerung an der Injektionsstelle eignet.

22. Verfahren zur Herstellung:
• von Medikamenten für die orale, nasale, vaginale, okulare, subkutane, intravenöse, intramuskuläre, intradermale, intraperitoneale oder intrazerebrale Verabreichung, wobei es sich bei den Wirkstoffen dieser Medikamente um Proteine, Glykoproteine, an eine oder mehrere Polyalkylenglykolketten gebundene Proteine, Peptide, Polysaccharide, Liposaccharide, Oligonukleotide, Polynukleotide und organische Nichtproteinmoleküle, die hydrophob, hydrophil oder amphiphil sein können, handeln kann;
• und/oder von Nährmitteln;
• und/oder von kosmetischen Produkten oder Pflanzenschutzprodukten;
**dadurch gekennzeichnet, daß** es im wesentlichen daraus besteht, daß man mindestens eine Polyaminosäure nach einem der Ansprüche 1 bis 12 und/oder die Zusammensetzung nach einem der Ansprüche 13 bis 21 einsetzt.
